# EUROPÄISCHE PATENTSCHRIFT

(11) **EP 2 181 654 B1**
(45) Veröffentlichungstag und Bekanntmachung des Hinweises auf die Patenterteilung: **12.08.2015**
(21) Anmeldenummer: 09173417.8
(22) Anmeldetag: 19.10.2009
(51) Int. Cl.: A61B 6/04, A61B 6/00, A61B 6/03

(54) **Tomosynthesegerät**
Tomosynthesis device
Appareil de tomosynthèse

(30) Priorität: 04.11.2008 DE 102008055737
(43) Veröffentlichungstag der Anmeldung: 05.05.2010
(73) Patentinhaber: Siemens Aktiengesellschaft, 80333 München (DE)
(72) Erfinder: Mertelmeier, Thomas, 91058, Erlangen (DE); Zhao, Wei, East Setauket, NY, 11733 (US)

(56) Entgegenhaltungen:
- EP-A- 1 004 274
- WO-A-01/66013
- WO-A-2004/043535
- US-A1- 2006 262 898
- US-A1- 2007 269 000

## Beschreibung

Die Erfindung betrifft ein Tomosynthesegerät mit einer Kompressions- und einer Lagerplatte.

Bei einer Tomosynthese handelt es sich um eine Untersuchung der Brust, insbesondere der weiblichen Brust, die vielfach mit dem Ziel durchgeführt wird, Tumore in einem möglichst frühen Stadium zu erkennen. Durch stetige Verbesserung der bildgebenden Verfahren wird angestrebt Bilder mit hoher Aussagekraft zu erzeugen, um mit hoher Sicherheit gutartige von bösartigen Veränderungen unterscheiden zu können. Ein Ziel bei der Verbesserung der bildgebenden Verfahren ist es, die Zahl der fehlerhaften Befunde, d.h. die Zahl der verdächtigen Befunde, die nicht von bösartigen Veränderung hervorgerufen sind, und die Zahl der nicht entdeckten Tumore, zu reduzieren.

Während der Tomosynthese wird die Brust in einem Tomosynthesegerät zwischen einer Lager- und einer Kompressionsplatte positioniert und komprimiert. Anschließend wird die Brust mit Hilfe einer Röntgenquelle aus verschiedenen Richtungen beleuchtet, wobei die einzelne, einen tomosynthetischen Bilddatensatz bildende Projektionen aufgenommen werden. Aus diesen Projektionen wird mit Hilfe eines Rekonstruktionsalgorithmus ein tomosynthetisches 3D-Röntgenbild errechnet.

Bei tomosynthetischen Untersuchung ist verschiedentlich beobachtet worden, dass die Brust nicht vollständig abgebildet wurde. Mit anderen Worten war es in manchen Fällen nicht möglich ein vollständiges tomosynthetisches 3D-Röntgenbild von der gesamten Brust zu erzeugen.

Aufgabe der vorliegenden Erfindung ist es, ein Tomosynthesegerät anzugeben, mit dem die tomosynthetische Untersuchung der Brust verbessert werden kann.

Die Aufgabe wird erfindungsgemäß durch ein Tomosynthesegerät mit den Merkmalen nach Anspruch 1 gelöst.

Ein solches Tomosynthesegerät umfasst eine Röntgenquelle, die zur Emission eines auf einen Detektor gerichteten Röntgenstrahlbündels geeignet ist, wobei im Strahlengang zwischen der Röntgenquelle und dem Detektor eine Kompressions- und eine Lagerplatte angeordnet sind. Zwischen der Kompressionsplatte und der Lagerplatte ist eine zu untersuchende Brust positionierbar und komprimierbar. Die Kompressionsplatte weist auf ihrer der Lagerplatte zugewandten Seite eine in Richtung der Lagerplatte geöffnete konkave Ausnehmung auf.

Der Konstruktion des erfindungsgemäßen Tomosynthesegerätes liegt die folgende Erkenntnis zu Grunde:

Konventionelle Tomosynthesegeräte verwenden Kompressionssysteme aus einer Lager- und einer Kompressionsplatte, die auch für die konventionelle Mammographie eingesetzt werden. Es wurde erkannt, dass im Vergleich zwischen Tomosynthese und Mammographie, vorausgesetzt die jeweils verwendeten Geräte besitzen die gleiche Aufnahmegeometrie, also insbesondere den gleichem Fokus-Detektor-Abstand, das der Tomosynthese zugängliche Untersuchungsvolumen kleiner ist als dasjenige Volumen, welches mit der herkömmlichen Mammographie untersucht werden kann. Unter einem Untersuchungsvolumen ist im Fall der Tomosynthese dasjenige Volumen zu verstehen, welches in allen Projektionen, die bei der tomosynthetischen Untersuchung aufgenommenen, vollständig abgebildet werden kann. Im Fall der konventionellen Mammographie ist das Untersuchungsvolumen dasjenige Volumen, welches von dem zur Aufnahme der Projektion verwendeten Röntgenstrahlbündel durchstrahlt wird.

Ausgehend von der Erkenntnis, dass Tomosynthese und Mammographie unterschiedlich große Untersuchungsvolumina aufweisen wurde außerdem erkannt, dass bei Kompressionssystem, wie sie für die konventionelle Mammographie eingesetzt werden, sofern diese Verwendung bei der Tomosynthese finden, ein Teil der Brust außerhalb des der tomosynthetischen Untersuchung zugänglichen Untersuchungsvolumens liegt.

Dieses Problem wird erfindungsgemäß durch den Einsatz einer Kompressionsplatte gelöst, die auf ihrer der Lagerplatte zugewandten Seite eine in Richtung der Lagerplatte geöffnete konkave Ausnehmung aufweist. Die zu untersuchende Brust wird von der konkaven Ausnehmung der Kompressionsplatte aufgenommen, wobei diese gezielt geformt wird. Bei der konventionellen Mammographie wird die Brust hauptsächlich mit Hilfe einer zentral, im Wesentlichen senkrecht zu der Detektoroberfläche wirkenden Kraft komprimiert. Die Brust wird vor allem in ihrem zentralen Bereich komprimiert. Im Gegensatz dazu wird nun die Brust auch mit Hilfe einer von den Seite auf diese einwirkenden Kraft komprimiert und positioniert. Dies führt dazu, dass das Brustgewebe von der Peripherie in einen zentralen Bereich der Lagerplatte bzw. der Kompressionsplatte gedrückt wird. Eine solche Formung und Kompression führt zu einer Verbesserung der tomosynthetischen Abbildung der Brust; die Wahrscheinlichkeit, dass ein Teil der Brust außerhalb des zugänglichen Untersuchungsvolumens liegt, ist wesentlich geringer.

Im Vergleich zu konventionellen Kompressionssystemen führt die Verwendung einer Kompressionsplatte mit einer konkaven Ausnehmung zu einer geringeren Kompression der Brust. Es wurde außerdem erkannt, dass dieser für die herkömmliche Mammographie nachteilige Effekt für die Tomosynthese weit weniger nachteilig ist oder sogar Vorteile mit sich bringt.

Bei der herkömmlichen Mammographie wird eine hohe Kompression der Brust angestrebt, um das Brustgewebe möglichst weit auseinander zu drücken, so dass im erzeugten Mammographiebild nach Möglichkeit eine Überlagerung einzelner Strukturen, die sich gegebenenfalls gegenseitig verdecken, vermieden werden kann. Dieses Problem tritt bei der Tomosynthese nicht auf, da aus den aufgenommenen einzelnen Projektionen ein tomosynthetisches 3D-Röntgenbild errechnet wird, in welchem auch in einer Richtung senkrecht zur Detektoroberfläche übereinander liegende Strukturen erkennbar sind.

Ein weiterer Grund für die bei der Mammographie angestrebte hohe Kompression der Brust ist die Tatsache, dass die im Mammographiebild auftretende Streustrahlung proportional zur Dicke des durchstrahlten Gewebes ist. Es wurde jedoch erkannt, dass der bei der Tomosynthese zur Rekonstruktion des tomosynthetischen 3D-Röntgenbildes eingesetzte Rekonstruktionsalgorithmus die in den Projektionen auftretende Streustrahlung effektiv kompensiert. Aus diesem Grund wirkt sich die aufgrund der geringeren Kompression größere Dicke des durchstrahlten Gewebes nicht negativ auf den Streustrahlungsanteil im resultierenden tomosynthetischen 3D-Röntgenbild aus.

Die mittels der Tomosynthese erzeugten tomosynthetischen 3D-Röntgenbilder weisen außerdem in einer Ebene parallel zu derjenigen des Detektors, üblicherweise wird für die Tomosynthese ein Flächendetektor verwendet, eine wesentlich höhere Auflösung als in einer Richtung senkrecht zu dieser auf. Eine geringere Kompression der Brust ist also auch aus diesem Grunde vorteilhaft. Veränderungen der Brust, beispielsweise Läsionen, die sich senkrecht zur Oberfläche des Detektors erstrecken, lassen sich auf diese Weise besser und sicherer detektieren. Außerdem wird die geringere Kompression von dem Patienten / der Patientin als weniger unangenehm empfunden.

Zusammenfassend kann durch den Einsatz einer Kompressionsplatte mit einer konkaven Ausnehmung die Brust in dasjenige Volumen gebracht werden, welches als zugängliches Untersuchungsvolumen bezeichnet wird und der tomosynthetischen Untersuchung zugänglich ist. Die im Vergleich zum Mammographie geringere Kompression der Brust wirkt sich bei der Tomosynthese, im Gegensatz zur Mammographie, nicht negativ aus.

Weitere vorteilhafte Ausgestaltungen des erfindungsgemäßen Tomosynthesegerätes gehen aus den von Anspruch 1 abhängigen Unteransprüchen hervor.

Im folgenden wird die Erfindung unter Bezugnahme auf die Figuren der Zeichnungen weiter erläutert. Dabei zeigen deren
- Fig. 1: und 3 je ein Tomosynthesegerät, in schematischer Frontalansicht und
- Fig. 2: und 4 je eine Kompressionsplatte, in schematischer Perspektivansicht.

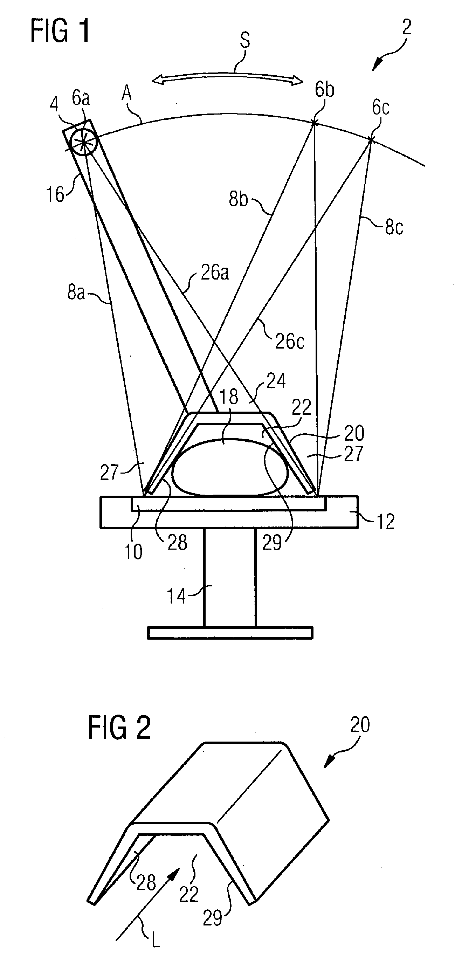

Fig. 1 zeigt ein Tomosynthesegerät 2 in einer schematischen Frontalansicht. Das Tomosynthesegerät 2 umfasst eine Röntgenquelle 4, von deren Fokus ein Röntgenstrahlbündel 8a..8c ausgeht, welches auf einen Flächendetektor 10 gerichtet ist, der in eine Lagerplatte 12 eingelassen ist. Eine Standsäule 14 trägt das Tomosynthesegerät 2.

Die Röntgenquelle 4 ist an einem Schwenkarm 16 befestigt, durch dessen Bewegung der Fokus der Röntgenquelle 4 in einem Abtastbereich, der üblicherweise zwischen 15° und 30° liegt, auf einer Abtastbahn A in einer Scanrichtung S bewegt werden kann. Fig. 1 zeigt beispielhaft drei verschiedene Fokuspositionen 6a, 6b und 6c, die der Fokus der Röntgenquelle 4 während eines Scanvorgangs annehmen kann. Ebenfalls beispielhaft zeigt Fig. 1 die Röntgenstrahlbündel 8a, 8b und 8c, die vom Fokus der Röntgenquelle 4 ausgehen, wenn dieser sich in der Fokusposition 6a, 6b bzw. 6c befindet. Der Fokus der Röntgenquelle 4 nimmt die Fokuspositionen 6a bzw. 6c ein, wenn sich die Röntgenquelle 4 in ihrer ersten bzw. zweiten Endposition des Abtastbereiches befindet.

Die Bewegung des Fokus der Röntgenquelle 4 ist nicht auf eine in Fig. 1 gezeigte Bahn, die näherungsweise dem Segment einer Kreisbahn entspricht, festgelegt. Alternativ kann die Bewegung des Fokus beispielsweise entlang einer Geraden erfolgen. Eine solche geradlinige Bewegung des Röntgenfokus kann zum einen durch die Verschiebung der Röntgenquelle 4 entlang eines entsprechenden Gestells erfolgen, zum anderen kann die Röntgenquelle 4 als sogenannte Multifokusröntgenquelle ausgestaltet sein, deren einzelne Emitter entlang der Scanrichtung S wie ein Lauflicht nacheinander durchgeschaltet werden, so dass auch in diesem Fall der Fokus der Röntgenquelle 4 entlang einer Geraden verschoben wird.

Eine auf der Lagerplatte 12 positionierte und komprimierte Brust 18, die als Untersuchungsobjekt dient, wird mit Röntgenstrahlbündeln 8a bis 8c aus verschiedenen Richtungen beleuchtet. Die Brust 18 wird zur Durchführung einer tomosynthetischen Untersuchung mit Hilfe einer Kompressionsplatte 20 zwischen dieser und der Lagerplatte 12 komprimiert und positioniert. Die Kompressionsplatte 20 weist auf ihrer der Lagerplatte 12 zugewandten Seite eine in Richtung der Lagerplatte 12 geöffnete konkave Ausnehmung 22 auf, in der die Brust 18 aufgenommen wird. Die Kompressionsplatte 20 bzw. deren Ausnehmung 22 ist vorzugsweise formstabil, d.h. die Form der Ausnehmung 22 ist im Belastungszustand, die Brust 18 ist komprimiert, und im Entlastungszustand, die Brust 18 ist nicht komprimiert, im Wesentlichen identisch. Die Form der in die Lagerplatte 20 eingelassenen konkaven Ausnehmung 22, genauer gesagt deren Innenkontur richtet sich im Wesentlichen nach der Form des mit Hilfe des Tomosynthesegerätes 2 zugänglichen Untersuchungsvolumens 24. Vorzugsweise ist die Form der Ausnehmung 22 so gewählt, dass diese vollständig innerhalb des der tomosynthetischen Untersuchung zugänglichen Untersuchungsvolumens (24) liegt.

Vorzugsweise wird die Kontur der der Lagerplatte 12 zugewandten Oberfläche, d.h. die Innenkontur der Ausnehmung 22 der Kompressionsplatte 20 an die äußere Kontur des zugänglichen Untersuchungsvolumens 24 angepasst. Dies wird am Verlauf der Flanken 28, 29 der in Fig. 1 gezeigten Kompressionsplatte 20 deutlich.

Das zugängliche Untersuchungsvolumen 24 ist dasjenige Volumen, welches von jeder der Projektionen erfasst wird, die während der Aufnahme eines tomosynthetischen Bilddatensatzes aufgenommen werden. Mit anderen Worten kann das zugängliche Untersuchungsvolumen allgemein wie folgt definiert werden. Die Röntgenquelle 4 des Tomosynthesegerätes 2, genauer gesagt der Fokus der Röntgenquelle 4 ist in einem Abtastbereich zwischen der ersten und einer zweiten Endposition bewegbar. Das zugängliche Untersuchungsvolumen 24 ist durch einen Überlappungsbereich zwischen einem ersten Röntgenstrahlbündel 8a, welches von der Röntgenquelle 4 in einer ersten Endposition aussendbar ist, und einem zweiten Röntgenstrahlbündel 8c, welches von der Röntgenquelle 4 in einer zweiten Endposition aussendbar ist, definiert.

In der für Fig. 1 gewählten schematischen Frontalansicht erscheinen die von den einzelne Fokuspositionen 6a..6c ausgehenden Röntgenstrahlbündel 8a..8c als Dreiecke. Es handelt sich dabei um Schnitte durch die Röntgenstrahlbündel 8a..8c, welche in der Realität näherungsweise kegelförmig sind. Das der tomosynthetischen Untersuchung zugängliche Untersuchungsvolumen 24 ist durch die Röntgenstrahlbündel 8a und 8c begrenzt, die von der Röntgenquelle 4 ausgehen, wenn sich diese in ihrer ersten, d.h. linken bzw. zweiten, d.h. rechten Endposition des Abtastbereiches befindet. Entsprechend wird das Untersuchungsvolumen 24 einerseits durch den rechten Grenzstrahl 26a des Röntgenstrahlbündels 8a und andererseits durch den linken Grenzstrahl 26c des Röntgenstrahlbündels 8c begrenzt.

Die Form der Kompressionsplatte 20 ist nun gerade so gewählt, dass sich die Brust 18 im komprimierten Zustand vollständig innerhalb des zugänglichen Untersuchungsvolumens 24 befindet. Anderenfalls bestünde die Gefahr, dass die Brust 18 in dem aufgenommenen tomosynthetischen 3D-Röntgenbild nicht vollständig abgebildet wird. Bei der Verwendung herkömmlicher Kompressionsplatten, wie sie aus der Mammographie bekannt sind, besteht insbesondere die Gefahr, dass ein Teil der Brust 18 in die nicht allen Projektionen, mit anderen Worten nicht allen während der Scanbewegung ausgesandten Röntgenstrahlbündeln 8a..8c zugänglichen Seitenbereiche 27 gedrückt wird. In diesen Seitenbereichen 27 wird das Brustgewebe lediglich von einigen, nicht aber von allen während der Scanbewegung ausgesandten Röntgenstrahlbündeln 8a..8c erfasst. Da der in den Seitenbereichen 27 gelegene Teil der Brust 18 nicht in allen aufgenommenen Projektionen abgebildet ist, kann von diesem Teil der Brust 18 kein vollständiges tomosynthetischen 3D-Röntgenbild rekonstruiert werden.

Die Form bzw. Kontur der Ausnehmung 22 kann unter Berücksichtigung der o.g. Bedingung, dass die Brust 18 im komprimierten Zustand vollständig innerhalb des zugänglichen Untersuchungsvolumens 24 liegen soll, prinzipiell frei gewählt werden. Bei dem in Fig. 1 dargestellten Ausführungsbeispiel ist die Oberflächenkontur der konkaven Ausnehmung 22 der Kompressionsplatte 20 so gewählt, dass diese zumindest teilweise der Form des zugänglichen Untersuchungsvolumens 24 entspricht. Dabei folgt die Innenkontur der Ausnehmung 22 im Bereich ihrer Flanken 28 bzw. 29 dem Verlauf der Grenzstrahlen 26a bzw. 26c, die das zugängliche Untersuchungsvolumen 24 begrenzen.

Wird nun die Brust 18 mit Hilfe der Kompressionsplatte 20 komprimiert, so wird die Brust von den Flanken 28, 29 der Kompressionsplatte 20 mit einer von der Seite auf diese einwirkenden Kraft beaufschlagt. Die Brust 18 wird in die Mitte des Flächendetektors 10 gedrückt, anstatt wie bei konventionellen Mammographiesystemen zur Seite.

Fig. 2 zeigt die aus Fig. 1 bekannte Kompressionsplatte 20 in einer schematischen Perspektivansicht. Die Kompressionsplatte 20 erstreckt sich in einer Längserstreckungsrichtung L nach der Art einer Rinne. Die Längserstreckungsrichtung L der Kompressionsplatte 20 ist senkrecht zu einer Brustwandebene orientiert, die wiederum senkrecht zu der Lagerplatte 12 (vgl. Fig. 1) steht. Das Tomosynthesegerät 2 ist beispielsweise zum Auflegen der Brust 18 auf die Lagerplatte 12 für eine Patientin von der Brustwandseite her zugänglich. In einer Schnittebene senkrecht zur Längserstreckungsrichtung L weist die Kompressionsplatte 20 eine konkave Innenkontur auf, die Form eines Polygonzuges aufweist.

Vorzugsweise ist die in Fig. 2 gezeigte Kompressionsplatte 20 unter Verzicht auf metallische Bauteile gefertigt, weiterhin vorzugsweise besteht diese vollständig aus einem Kunststoffmaterial. Metallische Bauteile, wie beispielsweise ein Rahmen oder Verstrebungen, können die Beleuchtung der Brust 18, im Rahmen der Durchführung einer tomosynthetischen Untersuchung, behindern. Der Verzicht auf metallische Bauteile ist daher vorteilhaft. Vorzugsweise ist die Kompressionsplatte aus Polycarbonat oder PET hergestellt. Die in Fig. 2 gezeigte Kompressionsplatte 20 ist vorzugsweise formstabil, d.h. ihre Form verändert sich zwischen einem Belastungszustand, in dem die Brust komprimiert ist, und einem Entlastungszustand, in dem die Brust entlastet ist, nur sehr unwesentlich.

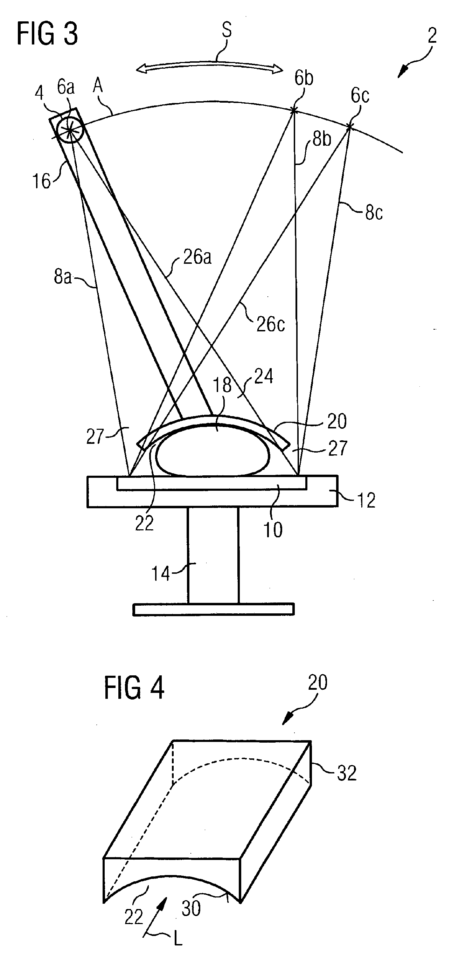

Fig. 3 zeigt ein weiteres Ausführungsbeispiel für ein Tomosynthesegerät 2. Dieses ist prinzipiell genauso aufgebaut wie das in Fig. 1 gezeigte Gerät, weist aber im Gegensatz zu diesem keine starre, formstabile sondern eine teilweise flexible Kompressionsplatte 20 auf. Deren konkave Ausnehmung 22 hat, betrachtet in einer Schnittebene senkrecht zu ihrer Längserstreckungsrichtung L einen abgerundeten Verlauf.

Fig. 4 zeigt eine perspektivische Detailansicht dieser Kompressionsplatte 20, mit welcher das in Fig. 3 gezeigte Tomosynthesegerät 2 ausgestattet ist. Die Kontur der Innenfläche 30 der Kompressionsplatte 20, die im montierten Zustand der Lagerplatte 12 zugewandt ist, entspricht im Wesentlichen einem Segment einer Zylindermantelfläche. Der der Zylindermantelfläche zu Grunde liegende Zylinder ist bei dem dargestellten Ausführungsbeispiel ein Kreiszylinder. Der dem Zylindermantel zugrunde liegende Zylinder kann jedoch ebenso ein parabolischer oder elliptischer Zylinder sein.

Die in Fig. 4 gezeigte Kompressionsplatte 20 ist reversibel also überwiegend elastisch verformbar, wobei diese sowohl im unbelasteten als auch um belasteten Zustand stets eine konkave Ausnehmung 22 aufweist. Die Kompressionsplatte 20 weist außerdem einen Rand 32 auf, welcher sich im montierten Zustand im Wesentlichen senkrecht zur Planebene der Lagerplatte 12 erstreckt, und sich vollumfänglich an die Innenfläche 30 der Kompressionsplatte 20 anschließt. Dieser Rand 32 dient der Verbesserung der Stabilität der Kompressionsplatte 20. Vorzugsweise wird die Kompressionsplatte 20 aus verschiedenen Materialien hergestellt, einem elastischen Material, beispielsweise einem röntgentransparenten Elastomer, auf ihrer der Brust 18 zugewandten Unterseite und einem starren/festen Material, beispielsweise Polycarbonat oder PET für den Rand 32. Auch die in Fig. 4 gezeigte Kompressionsplatte erstreckt sich rinnenförmig in einer senkrecht zur Brustwandseite orientierten Längserstreckungsrichtung L.

Wie bereits im Zusammenhang mit Fig. 1 erläutert, ist auch die in Fig. 4 gezeigte Kompressionsplatte 20 derart geformt, dass im komprimierten Zustand die Brust 18 vollständig innerhalb des zugänglichen Untersuchungsvolumens 24 liegt. Je nach Form und Größe der zu untersuchenden Brust 18 können die in den Fig. 1 und 3 gezeigten Tomosynthesegeräte 2 mit einer Kompressionsplatte 20 ausgerüstet werden, die eine Ausnehmung 22 aufweist, die der Form und Größe der Brust 18 angepasst ist. Dies trifft sowohl für die in Fig. 2 als auch für die in Fig. 4 gezeigte Kompressionsplatte 20 zu.

### Bezugszeichenliste

2 Tomosynthesegerät
4 Röntgenquelle
6a,b,c Fokusposition
8a,b,c Röntgenstrahlbündel
10 Flächendetektor
12 Lagerplatte
14 Standsäule
16 Schwenkarm
18 Brust
20 Kompressionsplatte
22 Ausnehmung
24 Untersuchungsvolumen
26a,26c Grenzstrahlen
27 Seitenbereich
28,29 Flanken
30 Innenfläche
32 Rand

L Längserstreckungsrichtung
S Scanrichtung
A Abtastbahn

## Patentansprüche

1. Tomosynthesegerät (2) mit einer Röntgenquelle (4) zur Emission eines auf einen Detektor (10) gerichteten Röntgenstrahlbündels (8a..8c), wobei im Strahlengang zwischen der Röntgenquelle (4) und dem Detektor (10) eine Kompressions- und eine Lagerplatte (20,12) angeordnet sind, zwischen denen eine zu untersuchende Brust (18) positionierbar und komprimierbar ist, und wobei die Kompressionsplatte (20) auf ihrer der Lagerplatte (12) zugewandten Seite eine in Richtung der Lagerplatte (12) geöffnete konkave Ausnehmung (22) aufweist, **dadurch gekennzeichnet daß**, die Kompressionsplatte (20) eine derart konkav geformte Ausnehmung (22) aufweist, dass die Brust (18) im komprimierten Zustand vollständig innerhalb eines einer tomosynthetischen Untersuchung zugänglichen Untersuchungsvolumens (24) liegt.

2. Tomosynthesegerät (2) nach Anspruch 1, wobei die Kompressionsplatte (20) formstabil ist.

3. Tomosynthesegerät (2) nach Anspruch 1, bei dem die Kompressionsplatte (20) zwischen einem unbelasteten und einem belasteten Zustand reversibel verformbar ist, wobei die Kompressionsplatte (20) sowohl im unbelasteten als auch im belasteten Zustand eine in Richtung der Lagerplatte (12) geöffnete konkave Ausnehmung (22) aufweist.

4. Tomosynthesegerät (2) nach einem der vorstehenden Ansprüche, wobei die Ausnehmung (22) der Kompressionsplatte (20) vollständig innerhalb eines einer tomosynthetischen Untersuchung zugänglichen Untersuchungsvolumens (24) liegt.

5. Tomosynthesegerät (2) nach einem der vorstehenden Ansprüche, bei dem die konkave Ausnehmung (22) der Kompressionsplatte (20) auf ihrer der Lagerplatte (12) zugewandten Innenseite eine Oberflächenkontur aufweist, die zumindest teilweise der Kontur eines zugänglichen Untersuchungsvolumens (24) entspricht.

6. Tomosynthesegerät (2) nach einem der vorstehenden Ansprüche, welches zur Durchführung einer Untersuchung von einer senkrecht zu der Lagerplatte (12) orientierten Brustwandebene her zugänglich ist, wobei die konkave Ausnehmung (22) der Kompressionsplatte (20) eine Längserstreckungsrichtung (L) aufweist, die senkrecht zu der Brustwandebene orientiert ist.

7. Tomosynthesegerät (2) nach Anspruch 6, wobei die Ausnehmung (22) der Kompressionsplatte (20) - betrachtet in einer Schnittebene senkrecht zu der Längserstreckungsrichtung (L) - eine konkave Innenkontur aufweist

8. Tomosynthesegerät (2) nach Anspruch 7, bei dem die der Lagerplatte (12) zugewandte Innenseite (30) der konkaven Ausnehmung (22) der Kompressionsplatte (20) - betrachtet in einer Schnittebene senkrecht zu der Längserstreckungsrichtung (L) - einen abgerundeten Verlauf aufweist.

9. Tomosynthesegerät (2) nach Anspruch 8, bei dem die Form der Innenseite (30) der konkaven Ausnehmung (22) im Wesentlichen einem Segment einer Zylindermantelfläche entspricht.

10. Tomosynthesegerät (2) nach Anspruch 9, bei dem der der Zylindermantelfläche zugrundeliegende Zylinder ein Kreiszylinder, ein parabolischer oder ein elliptischer Zylinder ist.

11. Tomosynthesegerät (2) nach Anspruch 7, bei dem die Innenkontur der konkaven Ausnehmung (22) der Kompressionsplatte (20) - betrachtet in einer Schnittebene senkrecht zu der Längserstreckungsrichtung (L) - die Form eines Polygonzuges aufweist.

12. Tomosynthesegerät (2) nach einem der Ansprüche 6 bis 11, bei dem die konkave Ausnehmung (22) der Kompressionsplatte (20) die Form einer sich in Längserstreckungsrichtung (L) erstreckenden Rinne aufweist.

13. Tomosynthesegerät (2) nach einem der vorstehenden Ansprüche, bei dem die Kompressionsplatte (20) einen im Wesentlichen senkrecht zu der Lagerplatte (12) orientierten Rand (32) aufweist.

14. Tomosynthesegerät (2) nach einem der vorstehenden Ansprüche, bei dem die Kompressionsplatte (20) unter Verzicht auf metallische Bauteile gefertigt ist.

15. Tomosynthesegerät (2) nach einem der vorstehenden Ansprüche, bei dem die Kompressionsplatte (20) vollständig aus einem Kunststoffmaterial gefertigt ist.

## Claims

1. Tomosynthesis device (2) comprising an x-ray source (4) for emitting an x-ray beam (8a..8c) directed onto a detector (10), wherein a compression plate and a support plate (20, 12) are arranged in the beam path between the x-ray source (4) and the detector (10), between which plates a breast (18) to be examined is positionable and compressible and wherein the compression plate (20) has a concave recess (22) that is open in the direction of the support plate (12) on the side thereof facing the support plate (12), **characterized in that** the compression plate (20) has such a concavely shaped recess (22) that, in the compressed state, the breast (18) lies completely within an examination volume (24) that is accessible to a tomosynthetic examination.

2. Tomosynthesis device (2) according to Claim 1, wherein the compression plate (20) is dimensionally stable.

3. Tomosynthesis device (2) according to Claim 1, in which the compression plate (20) is reversibly deformable between an unloaded and a loaded state, wherein the compression plate (20) has a concave recess (22) that is open in the direction of the support plate (12) in both the unloaded and loaded state.

4. Tomosynthesis device (2) according to one of the preceding claims, wherein the recess (22) of the compression plate (20) lies completely within an examination volume (24) that is accessible to a tomosynthetic examination.

5. Tomosynthesis device (2) according to one of the preceding claims, in which the concave recess (22) of the compression plate (20) has a surface contour on the inner side thereof facing the support plate (12), which contour at least partly corresponds to the contour of an accessible examination volume (24).

6. Tomosynthesis device (2) according to one of the preceding claims, which is accessible from a breast wall plane oriented perpendicular to the support plate (12) for carrying out an examination, wherein the concave recess (22) of the compression plate (20) has a direction of longitudinal extent (L), which is oriented perpendicular to the breast wall plane.

7. Tomosynthesis device (2) according to Claim 6, wherein the recess (22) of the compression plate (20) - as observed in a sectional plane perpendicular to the direction of longitudinal extent (L) - has a concave inner contour.

8. Tomosynthesis device (2) according to Claim 7, in which the inner side (30) of the concave recess (22) of the compression plate (20) facing the support plate (12) - as observed in a sectional plane perpendicular to the direction of longitudinal extent (L) - has a curved profile.

9. Tomosynthesis device (2) according to Claim 8, in which the shape of the inner side (30) of the concave recess (22) substantially corresponds to a segment of the lateral area of a cylinder.

10. Tomosynthesis device (2) according to Claim 9, in which the cylinder forming the basis of the lateral area of the cylinder is a circular cylinder, a parabolic or an elliptical cylinder.

11. Tomosynthesis device (2) according to Claim 7, in which the inner contour of the concave recess (22) of the compression plate (20) - as observed in a sectional plane perpendicular to the direction of longitudinal extent (L) - has the shape of a polygonal train.

12. Tomosynthesis device (2) according to one of Claims 6 to 11, in which the concave recess (22) of the compression plate (20) has the shape of a groove extending in the longitudinal direction of extent (L).

13. Tomosynthesis device (2) according to one of the preceding claims, in which the compression plate (20) has an edge (32) which is oriented substantially perpendicular to the support plate (12).

14. Tomosynthesis device (2) according to one of the preceding claims, in which the compression plate (20) is manufactured without the use of metallic components.

15. Tomosynthesis device (2) according to one of the preceding claims, in which the compression plate (20) is manufactured completely from a plastics material.

## Revendications

1. Appareil de tomosynthèse (2) comprenant une source de rayons X (4) pour l'émission d'un faisceau de rayons X (8a..8c) dirigé sur un détecteur (10), sur le trajet des rayons entre ladite source (4) et ledit détecteur (10) étant placées une plaque de compression et une plaque d'appui (20, 12) entre lesquelles un sein (18) devant être examiné peut être positionné et comprimé, et la plaque de compression (20) présentant sur son côté orienté vers la plaque d'appui (12) un évidement concave (22) ouvert en direction de la plaque d'appui (12), **caractérisé en ce que** la plaque de compression (20) présente un évidement concave (22) d'une forme telle que le sein (18), lorsqu'il est comprimé, se trouve entièrement à l'intérieur d'un volume d'examen (24) accessible pour un examen de tomosynthèse.

2. Appareil de tomosynthèse (2) selon la revendication 1, la plaque de compression (20) étant indéformable.

3. Appareil de tomosynthèse (2) selon la revendication 1, dans lequel la plaque de compression (20) est déformable de manière réversible entre un état non chargé et chargé, la plaque de compression (20) présentant tant à l'état non chargé que chargé un évidement concave (22) ouvert en direction de la plaque d'appui (12).

4. Appareil de tomosynthèse (2) selon l'une des revendications précédentes, l'évidement (22) de la plaque de compression (20) se trouvant entièrement à l'intérieur d'un volume d'examen (24) accessible pour un examen de tomosynthèse.

5. Appareil de tomosynthèse (2) selon l'une des revendications précédentes, dans lequel l'évidement concave (22) de la plaque de compression (20) présente sur son côté intérieur orienté vers la plaque d'appui (12) un contour de surface qui correspond au moins partiellement au contour d'un volume d'examen (24) accessible.

6. Appareil de tomosynthèse (2) selon l'une des revendications précédentes, qui pour réaliser un examen est accessible depuis un plan de paroi du sein orienté perpendiculairement à la plaque d'appui (12), l'évidement (22) de la plaque de compression (20) présentant une direction d'extension longitudinale (L) orientée perpendiculairement au plan de paroi du sein.

7. Appareil de tomosynthèse (2) selon la revendication 6, l'évidement (22) de la plaque de compression (20) - vu dans un plan de coupe perpendiculaire à la direction d'extension longitudinale (L) - présentant un contour intérieur concave.

8. Appareil de tomosynthèse (2) selon la revendication 7, dans lequel le côté intérieur (30) orienté vers la plaque d'appui (12) de l'évidement concave (22) de la plaque de compression (20) - vu dans un plan de coupe perpendiculaire à la direction d'extension longitudinale (L) - présente une allure arrondie.

9. Appareil de tomosynthèse (2) selon la revendication 8, dans lequel la forme du côté intérieur (30) de l'évidement concave (22) correspond sensiblement à un segment d'une surface d'enveloppe de cylindre.

10. Appareil de tomosynthèse (2) selon la revendication 9, dans lequel le cylindre à la base de la surface d'enveloppe de cylindre est un cylindre circulaire, un cylindre parabolique ou un cylindre elliptique.

11. Appareil de tomosynthèse (2) selon la revendication 7, dans lequel le contour intérieur de l'évidement concave (22) de la plaque de compression (20) - vu dans un plan de coupe perpendiculaire à la direction d'extension longitudinale (L) - présente la forme d'un tracé polygonal.

12. Appareil de tomosynthèse (2) selon l'une des revendications 6 à 11, dans lequel l'évidement concave (22) de la plaque de compression (20) présente la forme d'une rigole s'étendant en direction d'extension longitudinale (L).

13. Appareil de tomosynthèse (2) selon l'une des revendications précédentes, dans lequel la plaque de compression (20) présente un bord (32) orienté sensiblement perpendiculairement à la plaque d'appui (12).

14. Appareil de tomosynthèse (2) selon l'une des revendications précédentes, dans lequel la plaque de compression (20) est fabriquée en l'absence de composants métalliques.

15. Appareil de tomosynthèse (2) selon l'une des revendications précédentes, dans lequel la plaque de compression (20) est fabriquée entièrement en un matériau synthétique.
